# EUROPEAN PATENT SPECIFICATION

(11) **EP 2 823 756 B1**
(45) Date of publication and mention of the grant of the patent: **07.08.2019**
(21) Application number: 14173209.9
(22) Date of filing: 20.06.2014
(51) Int. Cl.: A61B 5/00

(54) **Object information acquiring apparatus and laser apparatus**
Objektinformationserfassungsvorrichtung und Laservorrichtung
Appareil d'acquisition d'informations d'objet et appareil à laser

(30) Priority: 09.07.2013 JP 2013143658
(43) Date of publication of application: 14.01.2015
(73) Proprietor: Canon Kabushiki Kaisha, Tokyo 146-8501 (JP)
(72) Inventor: Ichihara, Shigeru, Tokyo 146-8501 (JP); Kobayasho, Shuichi, Tokyo 146-8501 (JP)
(74) Representative: TBK

(56) References cited:
- JP-A- 2013 051 403
- JP-A- 2013 084 923
- US-B1- 6 193 711

## Description

### BACKGROUND OF THE INVENTION

### Field of the Invention

The present invention relates to an object information acquiring apparatus and a laser apparatus.

### Description of the Related Art

A technique known as photoacoustic tomography (PAT) is being developed. PAT is a technique for irradiating a measurement segment with pulsed laser light of the order of nanoseconds, receiving a photoacoustic wave generated in the measurement segment using a probe, and processing the resulting receive signal to form an image. PAT enables biological function analysis using spectral measurement based on the absorption coefficient of the biological tissue. For example, the oxidation state of hemoglobin in the blood is considered to be important biological function information.

S. Manohar et al. Proc. of SPIE vol. 6437 643702-1 reports a photoacoustic biological diagnosis apparatus in which a PAT apparatus is applied to observation of a tumor inside the breast. Peripheries of the tumor contain many new blood vessels extending from existing blood vessels. Thus, a method has been proposed which involves observing the state of the tumor by measuring the amount of hemoglobin contained in the new blood vessels and oxygen saturation corresponding to the oxidation state of the hemoglobin.

Laser light entering the living body diffuses and thus decays inside the living body. Thus, laser light with high laser power per pulse is needed to measure a photoacoustic wave generated in a relatively deep biological segment in a thick object such as the breast. A candidate for a light source for the photoacoustic biological diagnosis apparatus is a tunable laser using a titanium sapphire crystal or an alexandrite crystal as a laser medium. In particular, the alexandrite laser is suitable for the photoacoustic biological diagnosis apparatus because the alexandrite crystal, which is a laser medium, has a long fluorescence life and enables direct excitation using a flash lamp.

On the other hand, the flash lamp excited laser is disadvantageous in that output from the laser is likely to fluctuate as a result of degradation of the flash lamp or a change in environment such as the temperature around the laser. Japanese patent publication No. 4191539 discloses a method of controlling the output intensity of flash lamp output, which is an excitation source, by modifying laser output.
Patent Literature 1: Japanese Patent No. 4191539
Non Patent Literature 1: S. Manohar et al. Proc. of SPIE vol. 6437 643702-1

Prior art which is related to this field of technology can be found e.g. in document JP 2013-084923 A disclosing laser light source unit, and photoacoustic image producing device, and in document US 6,193,711 B1 disclosing rapid pulsed Er:YAG laser.

### SUMMARY OF THE INVENTION

For the photoacoustic biological diagnosis apparatus, it is important to make a comparison of the process of proliferation of tumor cells of the breast cancer or the like and transitional changes in the tumor state before and after chemotherapy. Thus, preferably, the photoacoustic biological diagnosis apparatus constantly and stably provides a photoacoustic signal that is dependent only on a change in the internal state of the living body.

In particular, photoacoustic signals resulting from laser light at two or more wavelengths are utilized in determining the oxygen saturation of the hemoglobin. Thus, keeping output intensity at each wavelength stable facilitates a comparison of the signal intensity and also enables a comparison of the oxygen saturation at a constantly stable detection accuracy.

The flash lamp excited laser has difficulty in keeping the laser output in a constant state. Japanese Patent No. 4191539 proposes a technique for stabilizing the output intensity by changing a voltage (or current) applied to the flash lamp. However, a change in thermal energy generated by the flash lamp changes the distribution of refractive index (thermal lens effect) in the laser medium. That is, a change in the voltage applied to the flash lamp fluctuates the thermal lens to make the output intensity unstable.

For the photoacoustic biological diagnosis apparatus, it is important to keep the output at two different wavelengths constant to maintain output stability. That is, a new challenge is to control the output so as to keep the thermal lens of the resonator constant at two different wavelengths.

The present invention has been made in view of the above-described problems. The present invention is developed to provide a laser apparatus that keeps the laser output at two different wavelengths stable with thermal stability maintained.

The present invention provides an object information acquiring and a laser apparatus as specified in the respective claims.

The present invention can provide a laser apparatus that keeps the laser output at two different wavelengths stable with thermal stability maintained.

Further features of the present invention will become apparent from the following description of exemplary embodiments with reference to the attached drawings.

### BRIEF DESCRIPTION OF THE DRAWINGS

FIG. 1 is a diagram showing a configuration of an embodiment of a photoacoustic biological diagnosis apparatus;
FIG. 2 is a diagram showing a configuration of an embodiment of a laser apparatus;
FIGS. 3A to 3C are diagrams illustrating control timings for the laser apparatus;
FIG. 4 is a diagram showing a configuration of an embodiment of the laser apparatus;
FIG. 5 is a diagram showing a configuration of an embodiment of the photoacoustic biological diagnosis apparatus;
FIG. 6 is a flowchart showing processing in a preparation step; and
FIG. 7 is a flowchart showing processing in photoacoustic measurement.

### DESCRIPTION OF THE EMBODIMENTS

Preferred embodiments of the present invention will be described below with reference to the drawings. The dimensions, materials, shapes, relative locations, and the like of components described below should be appropriately changed in accordance with the configuration of an apparatus to which the invention is applied and various conditions, and are not intended to limit the scope of the present invention to the description below.

An object information acquiring apparatus according to the present invention includes an apparatus that receives an acoustic wave generated, under a photoacoustic effect, in an object irradiated with light (electromagnetic wave) to acquire characteristic information as object information in the form of image data. The characteristic information acquired is indicative of the distribution of sources of the acoustic wave resulting from the light irradiation, the distribution of initial sound pressure in the object, or the distribution of optical energy absorption density or absorption coefficient derived from the distribution of initial sound pressure, or the distribution of concentrations of substances providing the tissue. Examples of the distribution of concentrations of substances include the distribution of oxygen saturation and the distribution of deoxyhemoglobin concentration.

The scope of the present invention is not limited to this, and the present invention is applicable to any apparatus using a mechanism that generates laser light as described below. For example, the present invention may be considered to be a laser apparatus that delivers light at different wavelengths (at least two wavelengths). A photoacoustic biological diagnosis apparatus will be described below as a typical example of an object information acquiring apparatus using a laser apparatus.

The present invention may further be considered as a method for controlling an object information acquiring apparatus using a photoacoustic effect or a method for controlling a laser apparatus.

The acoustic wave as referred to herein is typically an ultrasound wave and includes an elastic wave known as a sound wave, an ultrasound wave, or an acoustic wave. An acoustic wave generated under the photoacoustic effect in photoacoustic tomography is referred to as a photoacoustic wave or a photoacoustic ultrasound wave.

Furthermore, the following is referred to as a photoacoustic signal: an electric signal into which a photoacoustic wave is converted using a conversion element or a signal resulting from execution of signal processing (amplification or AD conversion) or information processing on the electric signal.

### (Configuration of the Apparatus)

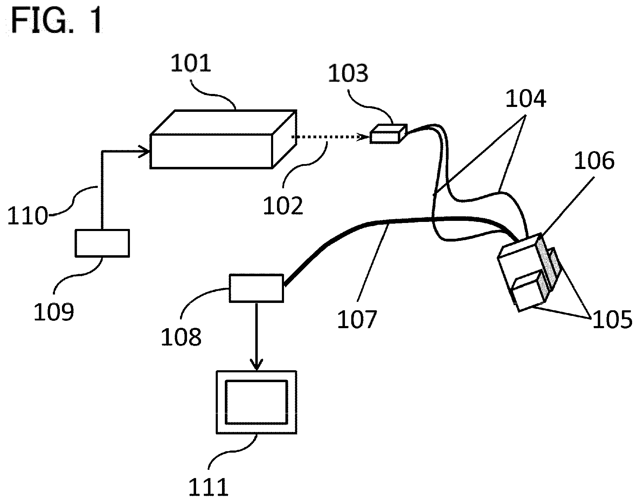

FIG. 1 is a schematic diagram of a photoacoustic biological diagnosis apparatus according to the present invention. The apparatus has an ultrasound probe 106 for detection of a photoacoustic wave and a laser apparatus 101 serving as a light source. A living body irradiation unit 105 and the ultrasound probe 106 are scanned over the living body to acquire a photoacoustic signal. A laser luminous flux resulting from oscillation of the light source is transmitted to the living body irradiation unit 105 via an optical transmission path 104 such as fibers. FIG. 1 shows a bundle fiber input end 103. In the figure, fiber transmission is illustrated, but space transmission utilizing an articulated arm or the like may be used.

A photoacoustic wave is detected by the ultrasound probe 106, and signal processing is executed on the photoacoustic wave by a processing apparatus 108 via a signal line 107. The processed photoacoustic wave is output to a monitor 111 as image information and biological information. Wavelength conversion and output control for the laser apparatus 101 are performed in accordance with a control signal 110 from a control apparatus 109. The control apparatus corresponds to a controller according to the present invention. The processing apparatus corresponds to a processor according to the present invention. The monitor corresponds to a display according to the present invention.

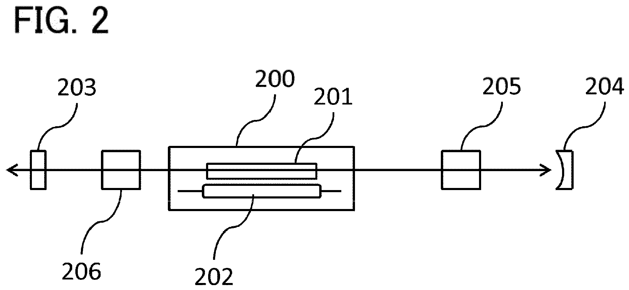

A tunable laser allowing wavelength to be varied is used as the laser apparatus. FIG. 2 is a diagram showing a configuration of the tunable laser. An output mirror 203 and a reflection mirror 204 provide a resonator. An excitation chamber 200, a Q switch 205, and a wavelength selection mechanism 206 are arranged inside the resonator. Inside the excitation chamber 200, a rod-like laser medium 201 and a flash lamp 202 exciting the laser medium 201 are arranged adjacently to each other. A portion of light emission energy of the flash lamp 202 is absorbed by the laser medium 201 to form an inverted distribution state. A loss in the resonator is reduced by the Q switch 205 at a desired timing to achieve laser oscillation. The laser medium 201 is a laser crystal with a high gain band. An oscillation wavelength for the laser medium 201 is selected by the wavelength selection mechanism 206.

### (Thermal Lens Effect)

For a laser using a flash lamp as an excitation source, the thermal lens needs to be kept constant to stabilize oscillation. The thermal lens will be described below in detail. Light emitted from the flash lamp mostly changes into heat to raise the temperature of the laser medium. As a result, a temperature distribution is generated inside the laser medium to change a local refractive index. The temperature of the laser medium depends on the temperature of circulating water around the laser medium and thermal energy resulting from light emission from the flash lamp. In general, the temperature varies between a central portion and a peripheral portion of the laser medium, and thus, the refractive index varies between the central portion and peripheral portion of the laser medium, causing a lens effect to be exerted. The lens effect is referred to as a thermal lens.

When a voltage (or a current) applied to the flash lamp - an excitation source - is changed to keep oscillation output at different wavelengths constant, the amount of heat changes, which causes a change in a thermal lens. This leads to variation in stable oscillation conditions. That is, when laser light with two different wavelengths is utilized for photoacoustic biological diagnosis, varying stability conditions are a problem.

### (Q Switch Control)

An embodiment of a method for laser control according to the present invention will be described.

A method will be described in which a Q switch operation timing is controlled in order to oscillate laser light with two different wavelengths constantly at a predetermined output level. The Q switch operation timing refers to the amount of time until a giant pulse oscillation is achieved by increasing the Q value of the resonator, based on light emission from the flash lamp. The Q switch corresponds to an oscillator according to the present invention.

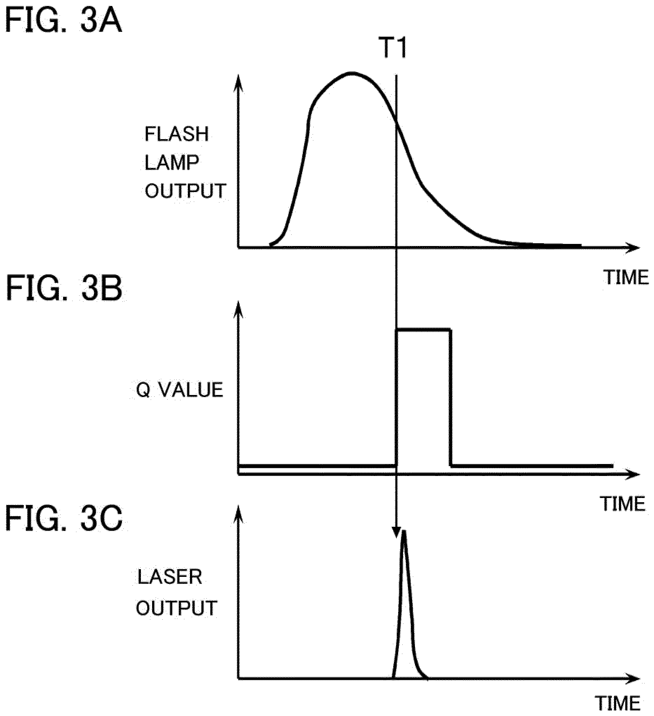

FIG. 3 shows relations between the flash lamp and Q value control using the Q switch and laser oscillation. FIG. 3A shows an output from the flash lamp. FIG. 3B shows timings for Q value control. FIG. 3C shows a laser output according to the Q value. In all graphs, the axis of abscissas indicates time.

The shape of a light emission pulse from the flash lamp, an excitation source, as shown in FIG. 3A depends on a pulse forming network (PFN) including a coil and a capacitor. When the pulse width of the flash lamp is equivalent to a fluorescence life at a high energy level in the laser crystal, the resultant laser system provides high output energy relative to input power and achieves oscillation efficiency. The Q switch is used to generate a giant pulse with high oscillation energy per pulse. Excitation starts, and then, with a sufficient high-energy-level inverted distribution density obtained, the loss in the resonator is reduced to increase the Q value of the resonator at a timing (T1) as shown in FIG. 3B. Thus, a giant pulse is oscillated as shown in FIG. 3C.

During this process, the high-energy-level inverted distribution density can be increased or reduced by changing the Q switch operation timing, that is, shifting the timing earlier or later with respect to T1. As a result, the laser oscillation output can be changed. The laser output control based on the operation timing control using the Q switch involves no change in thermal lens. Thus, for oscillation at two wavelengths with different gains, the oscillation output can be changed with the thermal lens kept constant.

A significant shift in the operation timing for the Q switch reduces oscillation efficiency, degrading laser characteristics in general. Furthermore, fluorescent oscillation making no contribution to laser oscillation increases the amount of spontaneous emission, which is subjected to stimulated amplification to become amplified spontaneous emission (ASE). This makes the laser output unstable. Additionally, when the operation timing for the Q switch is excessively early, high-energy-level inverted distribution and accumulation may occur again after the laser oscillation, resulting in double pulse oscillation.

Thus, when the output value is adjusted by changing the operation timing for the Q switch, it is important to avoid a significant reduction in efficiency with a margin for adjustment of an increase in output provided. Specifically, a laser output control range is preferably 80% to 100% of the maximum oscillation output. Furthermore, the adjustment of the output value is facilitated by setting an initialization value for the operation timing such that the value allows 90% of the maximum oscillation output to be achieved.

### (Thermal Equilibrium State)

The laser apparatus aligns the resonator so as to maximize the output in a thermal equilibrium state. However, a usage environment such as an external temperature and heat generation in the flash lamp affect the thermal state of the resonator, possibly making perfect maintenance of the thermal equilibrium state difficult in terms of practicality.

Furthermore, to keep the resonator thermally stable, the flash lamp preferably continues irradiation so as to bring the laser apparatus into a constant thermal state before measurement. However, when performing excessive irradiation before measurement, the flash lamp may be degraded and have its life shortened.

Furthermore, it is meaningful in a practical sense to utilize the laser apparatus in a quasi-equilibrium state similar to the thermal equilibrium state. An effective control method is operation timing control for the Q switch in which the laser output from the laser apparatus in the quasi-equilibrium state is kept constant.

### (Voltage Adjustment for the Flash Lamp)

When the use of the photoacoustic biological diagnosis apparatus in a constant state over a long period is taken into account, reduced laser output resulting from degradation of the flash lamp needs to be dealt with. When the flash lamp is exhausted and degraded, the output intensity may decrease by 30% or more. The exhaustion of the flash lamp changes the thermal state in the resonator. Dealing with such a significant fluctuation in output needs not only the output adjustment based on the operation timing for the Q switch but also adjustment of a voltage applied to the flash lamp by a power supply. A regulator that adjusts the voltage may be a controller for the laser apparatus or a dedicated circuit or information processing apparatus.

An embodiment including a process of adjusting the voltage applied to the flash lamp will be described. In this case, the voltage applied to the flash lamp is determined during a preparation step before biological measurement and not changed during the biological measurement.

First, the wavelength selection mechanism selects one of the wavelengths. The wavelength selection mechanism corresponds to a selector according to the present invention.

Then, the flash lamp is warmed up until the resonator is brought into the thermal equilibrium state, and output energy is measured. If a predetermined output fails to be obtained at the voltage applied to the flash lamp for initialization, that is, if the flash lamp is degraded, the applied voltage is increased so as to compensate for a decrease in energy, to provide predetermined output energy. The wavelengths used are not limited, but the applied voltage is determined by constantly using the same wavelengths.

The life of the flash lamp depends on the configuration of the laser resonator and generally corresponds to approximately 10 million shots. According to the present invention, the end of the life of the flash lamp is defined to be a state where, with the same voltage kept applied to the flash lamp, the output from the flash lamp has decreased by 30% compared to the initial output. When the photoacoustic biological diagnosis apparatus is utilized, the flash lamp is basically not significantly degraded in a short period of time such as several days. However, the life of the flash lamp varies among individual flash lamps, and thus, the flash lamp is preferably constantly checked for its state during the preparation step before biological measurement.

### (Characteristics of the Laser Medium)

A titanium sapphire laser and an alexandrite laser are examples of a tunable pulse laser that excites the flash lamp, a light source for the photoacoustic biological diagnosis apparatus. The tunable pulse laser has an oscillation wavelength band matching an absorption band for oxyhemoglobin and deoxyhemoglobin and is thus a preferable light source for detection of blood oxygen saturation.

To allow the oxygen saturation to be constantly measured under the same conditions, energy output at two different wavelengths needs to be constantly kept stable. The quantity of output energy need not be the same for the different outputs. However, the resultant photoacoustic signal output and thus the accuracy of the resultant oxygen saturation decrease consistently with the laser output. On the other hand, the irradiation energy intensity of the laser output is preferably maximized within a possible range, with irradiation safety for the living body maintained. Hence, preferably, approximately the same output is provided at the two wavelengths, and the respective quantities of output energy are kept stable.

The alexandrite laser has the advantages of being easily excited by the flash lamp, needing only low costs, and enabling oscillation output with high output energy per pulse. On the other hand, the gain of the alexandrite laser crystal depends on the wavelength, and differs greatly between a wavelength of 750 nm and a wavelength of 800 nm, which are appropriate for measurement of the oxidation state of hemoglobin. When the laser crystal has a high gain, the resonator needs to be designed such that a loss in the resonator is controlled, with the balance between the gain and loss being taken into account, in order to maintain the oscillation output at two different wavelengths stably.

As such design of the resonator, the design of the refractive index of the output mirror is particularly effective. When the refractive index is changed in accordance with the wavelength, the loss changes. This enables the laser output to be made more constant without changing the voltage applied to the flash lamp for each wavelength. Such design of the resonator enables the difference in output between the wavelengths to be reduced to about 15%. On the other hand, the refractive index of the output mirror undergoes a manufacturing variation among lots. Thus, completely eliminating the difference in output between the wavelengths is difficult. Moreover, the gain of the laser crystal also undergoes a manufacturing variation. Thus, the characteristics of the laser apparatus vary among individual laser apparatuses within the range of design.

The state where the output at two different wavelengths is stabilized as described herein refers to the ratio of outputs at two different wavelengths being maintained constant. Furthermore, the ratio of the quantities of output energy at the respective wavelengths is preferably 90% or more and more preferably 95% or more. In other words, control is performed such that the value of the output energy at one of a plurality of wavelengths which involves the smallest output energy value is 90% or more of the value of output energy at one of the plurality of wavelengths which involves the largest output energy value.

In this case, the output of the laser varies in accordance with the pulse, and thus, the output value is preferably the average value for a given pulse train determined with an output variation according to the pulse taken into account.

### <Example 1>

An example of the present invention will be illustrated. In this case, an example of a method for controlling a flash lamp excited pulse laser will be illustrated.

### (Apparatus Configuration)

In Example 1, an alexandrite laser was used as a light source. However, the light source used in the present invention is not limited to the alexandrite.

The laser apparatus is configured such that a wavelength of 795 nm and a wavelength of 755 mm oscillate. Repetition frequency is 20 Hz.

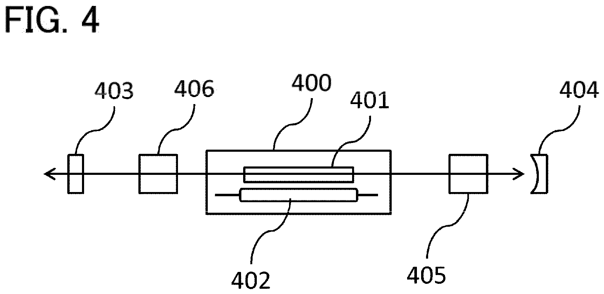

FIG. 4 shows a configuration of a resonator including an alexandrite laser. A flash lamp 402 that excites alexandrite and an alexandrite crystal 401 are arranged in an excitation chamber 400. The alexandrite crystal is immersed in circulating water at 75°C. An optical thin film is applied to an output mirror 403 so that reflectance is 72% for the wavelength of 795 nm and 42% for the wavelength of 755 nm. A dielectric reflection film that significantly reflects a center wavelength of 750 nm to 800 nm is applied to a reflection film 404. A Q switch 405 including a Pockels cell is located on an optical axis between the output mirror 403 and the excitation chamber 400. A wavelength selection mechanism 406 is a birefringence filter.

### (Process Flow)

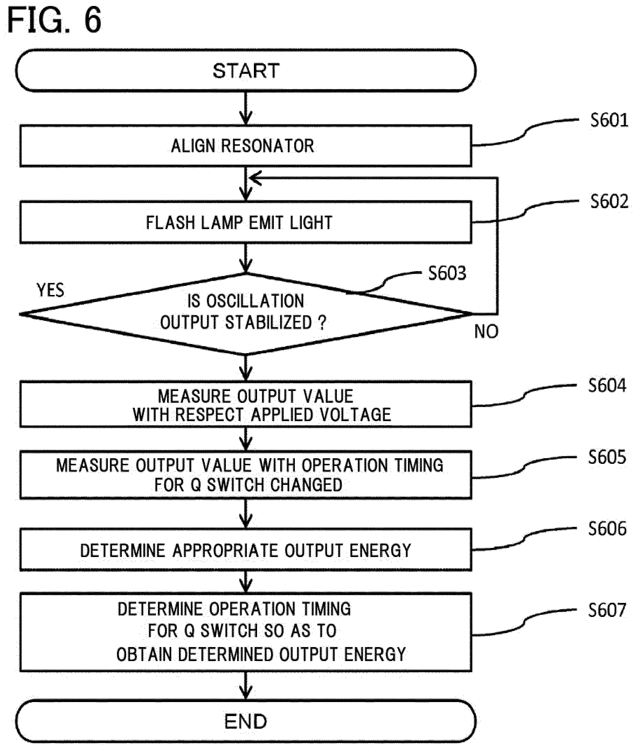

Now, a control method will be described in which characteristic information on an object are acquired using the laser apparatus in FIG. 4. In Example 1, particularly the preparation step will be described in detail with reference to a flowchart in FIG. 6.

For the initialization value for the laser apparatus, the resonator was aligned in a thermally stable state (S601).

Subsequently, the oscillation output was measured to determine a set value. A warm-up step was executed by allowing the flash lamp to emit light for a predetermined period of time until thermal stability is achieved so as to stabilize the oscillation output at two wavelengths of 755 nm and 795nm (S602) . A condition for the state with stable oscillation outputs is that the average output of a pulse train is constant for the voltage applied to the flash lamp. A smaller number of laser pulses contained in the pulse train increases the output variation among pulses, and an excessively large number of pulses need much measurement time. Hence, the number of measurements of the average output is preferably about 100 to 1,000. In Example 1, the number of pulses is 200.

Once the oscillation output is stabilized (S603 = YES), the output value is measured (S604) . The maximum output resulting from an applied voltage of 1 kV was 215 mJ at a wavelength of 755 nm and 190 mJ at a wavelength of 795 nm.

The thermal state of the resonator depends on the applied voltage. Then, a change in laser output was measured with the voltage applied to the flash lamp fixed to 1 kV and with the operation timing for the Q switch varied (S605). The resonator used in Example 1 provides the maximum output when the operation timing for the Q switch is 160 us. Furthermore, the output energy decreases from the maximum output when the operation timing corresponds to a length of time of approximately 160 us. When the operation timing corresponds to a length of time equal to or shorter than 160 us, the laser output intensity is low because the high-energy-level inverted distribution density of the alexandrite crystal is in an unsaturated state. On the other hand, when the operation timing corresponds to a length of time equal to or longer than 160 us, the laser output intensity deceases because the temporarily accumulated high-energy-level inverted distribution density is likely to decrease due to fluorescent spontaneous emission.

With these characteristics taken into account, the operation timing for the Q switch was controlled without significantly reducing efficiency, to determine the value of laser output as an initialization value (S606). That is, the oscillation output values for the photoacoustic biological diagnosis apparatus were determined to be an output energy of 188 mJ at the wavelength of 755 nm and an output energy of 180 mJ at the wavelength of 795 nm. In this case, the ratio of the quantities of output energy at the respective wavelengths is approximately 96%, which is indicative of a suitable range.

The range of the operation timing for the Q switch resulting in output energy equal to 80% to 100% of the maximum output was 130 us to 190 us. Thus, in Example 1, the operation timing for the Q switch used for output control was set to 130 us to 160 us (S607) .

Such settings as in Example 1 enables the thermal state based on the lamp emission energy to be maintained constant regardless of the wavelength because the lamp energy is constant at 1 kV. Furthermore, the present invention changes the operation timing for the Q switch in accordance with the exhaustion of the lamp energy or a change in the thermal state in the resonator. This enables the maintenance of the initialization values, that is, the output energy of 188 mJ at the wavelength of 755 nm and the output energy of 180 mJ at the wavelength of 795 nm, allowing the output energy to be kept stable. When the oxygen saturation was measured using a biological simulation sample (phantom) with an absorbent imbedded therein and simulating a blood vessel equivalent to an oxygen saturation of 80%, the oxygen saturation was able to be constantly stably calculated at a high accuracy.

### <Example 2>

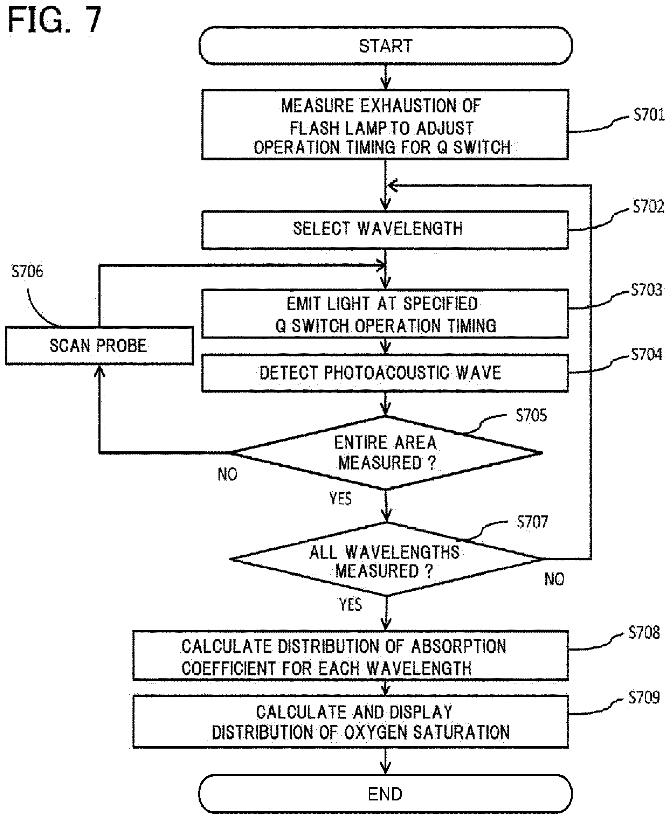

In Example 2, a photoacoustic measurement procedure will be illustrated which uses a flash lamp excited laser apparatus. In Example 2, particularly the measurement step will be described in detail with reference to a flowchart in FIG. 7.

### (Apparatus Configuration)

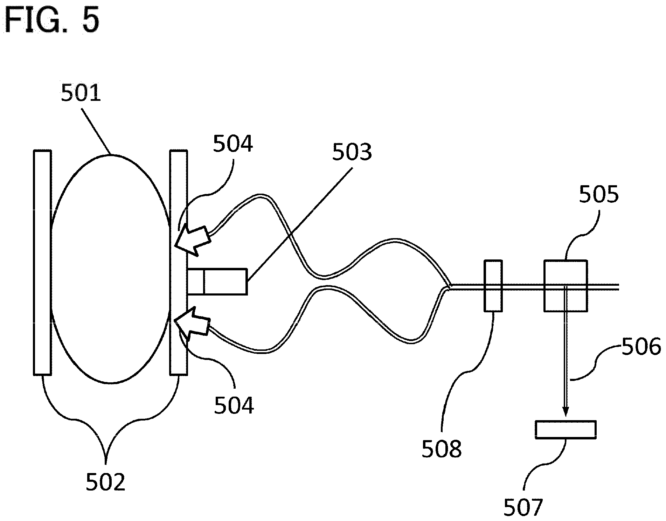

An apparatus in FIG. 5 was used as a photoacoustic biological diagnosis apparatus. This apparatus can measures a relatively large biological segment such as the breast. In Example 2, instead of the living body, a phantom 501 was measured which included an absorbent embedded therein and simulating a blood vessel equivalent to an oxygen saturation of 80%.

The phantom 501 is lightly pressed between approximately parallel flat plates 502, to fix a measurement segment. Illumination units 504 are provided at the respective opposite sides of an ultrasound probe 503 so as to serve as output ends for laser light. Measurement is performed with a scanner, not shown in figures, by simultaneously scanning the ultrasound probe 503 and the illumination unit 504 with respect to the flat plates 502. The illumination units 504 may be arranged opposite the probe across the object. The illumination units correspond to an illuminator according to the present invention.

The scanning of the ultrasound probe was based on one-stripe reciprocating scanning. Depending on a size of a measurement segment, stripe scanning was performed along a plurality of lines to measure the entire measurement segment. In the one-stripe reciprocating scanning, photoacoustic measurement was performed by delivering laser light with a wavelength of 755 nm during a forward scan, while delivering laser light with a wavelength of 795 nm during a backward scan. That is, the measurement was preformed with one of the two wavelengths alternately selected. The number of laser irradiations in one direction on one line corresponds to a pulse train according to Example 2.

The stripe refers to an area from which data is acquired when the object is linearly scanned using the ultrasound probe. An ultrasound probe with a plurality of conversion elements arranged in at least one direction is preferably scanned orthogonally to the direction in which the conversion elements are arranged because this operation allows a large area to be measured at a time.

Laser output was measured by branching a luminous flux not having impinged on the phantom yet into two luminous fluxes using a luminous flux branch member 505 and measuring the resultant branching luminous flux 506 using an energy detector 507. The branching luminous flux, which was equal to approximately 2% of the total luminous flux, was monitored using the energy detector. The amount of light delivered to the phantom was converted using the branch ratio.

In the preparation step, the set initialization values are such that a voltage of 1 kV is applied to the flash lamp and that an energy of 188 mJ and an energy of 180 mJ are output at a wavelength of 755 nm and at a wavelength of 795 nm, respectively, based on the operation timing control for the Q switch.

### (Process Flow)

In the preparation step before measurement, the output at each wavelength was measured with irradiation of the phantom 501 shielded by a shield 508. The average value for a pulse train needed for one stripe for the photoacoustic measurement was determined as the output value obtained in a measurement start state (before the phantom was irradiated). The output obtained in the measurement start state depends on the thermally stable state of the resonator. However, in Example 2, the output was 180 mJ at 755 nm and 170 mJ at 795 nm. At the beginning of the scan for the first stripe, the operation timing for the Q switch started to be controlled so as to obtain an output of 188 mJ at a wavelength of 755 nm and an output of 180 mJ at a wavelength of 795 nm. This process may be executed in accordance with a procedure similar to the procedure described above in Example 1.

The set values acquired in the preparation step were stored in a memory (not shown in the drawings), and then, measurement of the phantom was started. This process will be described with reference to a flowchart in FIG. 7.

Before detection of a photoacoustic wave, the flash lamp may be checked for exhaustion and the voltage may be adjusted in accordance with the degree of the exhaustion as described above (S701). This allows the accuracy of the measurement to be increased with degradation of the flash lamp taken into account.

Subsequently, the average output of a pulse train at each wavelength for each stripe was calculated, and the operation timing for the Q switch was controlled for each pulse train so as to make the average output value of the pulse train at each wavelength constant.

In specific measurements, the wavelength is selected (S702), and light is emitted at the selected wavelength at a specified operation timing for the Q switch (S703). Then, a predetermined quantity of output energy is obtained. Then, a photoacoustic wave generated in the object is detected using the probe (S704).

Until the entire measurement target area is measured (S705 = YES), the probe is scanned (S706) to continue detection of a photoacoustic wave. This process is executed with the wavelength and the operation timing for the Q switch changed (S707). The scanning may be performed by changing the wavelength between the forward scan and the backward scan on the stripe instead of measuring the entire area at each wavelength.

Then, the distribution of the absorption coefficient is calculated based on the resultant photoacoustic signal (S708) . The distribution of the oxygen saturation can then be calculated and displayed by executing a known calculation process (S709).

In Example 2, the output energy at each wavelength was stable all over the area on which the phantom was scanned. Thus, the irradiation conditions were maintained to allow a stable photoacoustic signal to be obtained. The oxygen saturation calculated from the resultant photoacoustic signal was accurate and was restrained from varying all over the measurement area.

When the above-described measurement was repeatedly performed during several days, the photoacoustic signal and a variation in the accuracy of the oxygen saturation were found to be constant.

As described above, the preset invention can keep the laser output at two different wavelengths stable with thermal stability maintained, regardless of a loss in the resonator, the gain of the laser medium, and a change in laser oscillation efficiency resulting from a change in the efficiency of excitation performed by the flash lamp.

That is, the present invention can provide a method for controlling a laser apparatus for use in a photoacoustic biological diagnosis apparatus so that the oscillation output at two different wavelengths can be stabilized.

While the present invention has been described with reference to exemplary embodiments, it is to be understood that the invention is not limited to the disclosed exemplary embodiments but by the scope of the following claims.

## Claims

1. A laser apparatus comprising:
a resonator including at least a laser medium (201), a flash lamp (202) configured to excite the laser medium (201), and an output mirror (203) configured to output laser light;
an oscillator configured to oscillate the laser light;
a controller (109) configured to control a timing at which the oscillator oscillates the laser light after excitation performed by the flash lamp (202) is started; and
a selector (206) configured to select a wavelength of the laser light from a plurality of wavelengths;
**characterised in that** the controller changes the timing, at which the laser light is oscillated, for each of the wavelengths in such a manner that a difference in output energy of the laser light between the plurality of wavelengths falls within a predetermined range.

2. The laser apparatus according to claim 1, wherein the controller performs control in such a manner that, at each wavelength, the output energy of the laser light is 80% to 100% of a maximum output at a wavelength.

3. The laser apparatus according to claim 2, wherein the controller performs control in such a manner that, at each wavelength, the output energy of the laser light is 90% of a maximum output at a wavelength.

4. The laser apparatus according to any one of claims 1 to 3, wherein the controller performs control in such a manner that an output energy value at a wavelength that, from among the plurality of wavelengths, exhibits a smallest output energy value is 90% or more of an output energy value at a wavelength that, from among the plurality of wavelengths, exhibits a largest output energy value.

5. The laser apparatus according to claim 4, wherein the controller performs control in such a manner that the output energy value at the wavelength that, from among the plurality of wavelengths, exhibits the smallest output energy value is 95% or more of the output energy value at the wavelength that, from among the plurality of wavelengths, exhibits the largest output energy value.

6. The laser apparatus according to any one of claims 1 to 5, further comprising a regulator configured to adjust and maintain at a predetermined level a voltage applied to the flash lamp while the object is being irradiated with the laser light.

7. The laser apparatus according to claim 6, wherein the regulator measures the output energy of the flash lamp before the object is irradiated with the laser light, and increases the applied voltage when predetermined output energy fails to be obtained.

8. The laser apparatus according to any one of claims 1 to 7, wherein the output mirror has a light reflectance varying in accordance with the wavelength of the laser light.

9. The laser apparatus according to any one of claims 1 to 8, wherein the oscillator is a Q switch.

10. An object information acquiring apparatus comprising:
the laser apparatus according to any one of claims 1 to 9,
a probe (106) configured to detect an acoustic wave generated in an object irradiated with the laser light; and
a processor (108) configured to acquire characteristic information on the object by using the acoustic wave.

11. The object information acquiring apparatus according to claim 10, wherein
the selector selects the wavelength of the laser light from two wavelengths, and
the processor calculates an oxygen saturation of the object by using the characteristic information obtained at each of the two wavelengths.

12. The object information acquiring apparatus according to claim 11, wherein the two wavelengths are 750 nm and 800 nm.

13. The object information acquiring apparatus according to any one of claims 10 to 12, further comprising:
an illuminator that irradiates the object with the laser light; and
a scanner that reciprocates the illuminator over a stripe on the object,
wherein the selector selects different wavelengths for a forward scan and for a backward scan within the stripe.

14. The object information acquiring apparatus according to any one of claims 10 to 13, further comprising a display that displays characteristic information on the object.

## Patentansprüche

1. Laservorrichtung mit:
einem Resonator, der zumindest ein Lasermedium (201), eine Blitzlampe (202), die konfiguriert ist, das Lasermedium (201) anzuregen, und einen Ausgabespiegel (203), der konfiguriert ist, ein Laserlicht auszugeben, enthält;
einem Oszillator, der konfiguriert ist, das Laserlicht zu oszillieren;
einer Steuerungseinrichtung (109), die konfiguriert ist, einen Zeitpunkt, zu dem der Oszillator das Laserlicht oszilliert, nachdem eine Anregung, die durch die Blitzlampe (202) durchgeführt wird, gestartet wurde, zu steuern; und
einer Auswahleinrichtung (206), die konfiguriert ist, eine Wellenlänge des Laserlichts aus einer Vielzahl von Wellenlängen auszuwählen;
**dadurch gekennzeichnet, dass**
die Steuerungseinrichtung den Zeitpunkt, zu dem das Laserlicht oszilliert wird, für jede der Wellenlängen in solch einer Weise ändert, dass eine Differenz in einer Ausgabeenergie des Laserlichts zwischen der Vielzahl der Wellenlängen in einen vorbestimmten Bereich fällt.

2. Laservorrichtung nach Anspruch 1, wobei die Steuerungseinrichtung eine Steuerung in solch einer Weise durchführt, dass zu jeder Wellenlänge die Ausgabeenergie des Laserlichts 80% bis 100% einer maximalen Ausgabe bei einer Wellenlänge ist.

3. Laservorrichtung Anspruch 2, wobei die Steuerungseinrichtung eine Steuerung in solch einer Weise durchführt, dass bei jeder Wellenlänge die Ausgabeenergie des Laserlichts 90% einer maximalen Ausgabe bei einer Wellenlänge ist.

4. Laservorrichtung nach einem der Ansprüche 1 bis 3, wobei die Steuerungseinrichtung eine Steuerung auf solch eine Weise durchführt, dass ein Ausgabeenergiewert bei einer Wellenlänge, die unter der Vielzahl der Wellenlängen einen kleinsten Ausgabeenergiewert annimmt, 90% oder mehr eines Ausgabeenergiewert bei einer Wellenlänge annimmt, die unter der Vielzahl der Wellenlängen einen größten Ausgabeenergiewert annimmt.

5. Laservorrichtung nach Anspruch 4, wobei die Steuerungseinrichtung eine Steuerung auf solch eine Weise durchführt, dass der Ausgabeenergiewert bei der Wellenlänge, die unter der Vielzahl der Wellenlängen den kleinsten Ausgabeenergiewert annimmt, 95% oder mehr des Ausgabeenergiewerts bei der Wellenlänge ist, die unter der Vielzahl der Wellenlängen den größten Ausgabeenergiewert annimmt.

6. Laservorrichtung nach einem der Ansprüche 1 bis 5, ferner mit einem Regulator, der konfiguriert ist, eine Spannung, die an die Blitzlampe angelegt wird, während das Objekt mit dem Laserlicht bestrahlt wird, anzupassen und auf einem vorbestimmten Niveau beizubehalten.

7. Laservorrichtung nach Anspruch 6, wobei der Regulator die Ausgabeenergie der Blitzlampe misst, bevor das Objekt mit dem Laserlicht bestrahlt wird, und die angelegte Spannung erhöht, wenn eine vorbestimmte Ausgabeenergie nicht erhalten wird.

8. Laservorrichtung nach einem der Ansprüche 1 bis 7, wobei der Ausgabespiegel eine Lichtreflektanz hat, die gemäß der Wellenlänge des Laserlichts variiert.

9. Laservorrichtung nach einem der Ansprüche 1 bis 8, wobei der Oszillator ein Q-Switch ist.

10. Objektinformationserhaltevorrichtung mit:
der Laservorrichtung nach einem der Ansprüche 1 bis 9,
einem Meßkopf (106), der konfiguriert ist, eine akustische Welle, die in dem Objekt, das mit dem Laserlicht bestrahlt wird, erzeugt wird, zu erfassen; und
einem Prozessor (108), der konfiguriert ist, eine charakteristische Information über das Objekt unter Verwendung der akustischen Welle zu erhalten.

11. Objektinformationserhaltevorrichtung nach Anspruch 10, wobei
die Auswahleinrichtung die Wellenlänge des Laserlichts aus zwei Wellenlängen auswählt, und
der Prozessor eine Sauerstoffsättigung des Objekts unter Verwendung der charakteristischen Information, die bei jeder der zwei Wellenlängen erhalten wird, berechnet.

12. Objektinformationserhaltevorrichtung nach Anspruch 11, wobei die zwei Wellenlängen 750 nm und 800 nm sind.

13. Objektinformationserhaltevorrichtung nach einem der Ansprüche 10 bis 12, ferner mit:
einer Beleuchtungseinrichtung, die das Objekt mit dem Laserlicht bestrahlt; und
einem Scanner, der die Beleuchtungseinrichtung über einen Streifen auf dem Objekt hin und her bewegt,
wobei die Auswahleinrichtung verschiedene Wellenlängen für ein Vorwärtsabtasten und für ein Rückwärtsabtasten innerhalb des Streifens auswählt.

14. Objektinformationserhaltevorrichtung nach einem der Ansprüche 10 bis 13, ferner mit einer Anzeigevorrichtung, die eine charakteristische Information über das Objekt anzeigt.

## Revendications

1. Appareil laser, comprenant :
un résonateur comprenant au moins un milieu actif (201), une lampe à éclair (202) configurée pour exciter le milieu actif (201), et un miroir de sortie (203) configuré pour sortir de la lumière laser ;
un oscillateur configuré pour faire osciller la lumière laser ;
un organe de commande (109) configuré pour commander une temporisation à laquelle l'oscillateur fait osciller la lumière laser après le début de l'excitation assurée par la lampe à éclair (202) ; et
un sélecteur (206) configuré pour sélectionner une longueur d'onde de la lumière laser parmi une pluralité de longueurs d'onde ;
**caractérisé en ce que**
l'organe de commande modifie la temporisation, à laquelle la lumière laser est amenée à osciller, pour chacune des longueurs d'onde de sorte qu'une différence d'énergie de sortie de la lumière laser entre la pluralité de longueurs d'onde s'inscrive dans une plage prédéterminée.

2. Appareil laser selon la revendication 1, dans lequel l'organe de commande effectue une commande telle que, à chaque longueur d'onde, l'énergie de sortie de la lumière laser s'inscrive dans la plage de 80 % à 100 % d'une sortie maximale à une longueur d'onde.

3. Appareil laser selon la revendication 2, dans lequel l'organe de commande effectue une commande telle que, à chaque longueur d'onde, l'énergie de sortie de la lumière laser soit de 90 % d'une sortie maximale à une longueur d'onde.

4. Appareil laser selon l'une quelconque des revendications 1 à 3, dans lequel l'organe de commande effectue une commande telle qu'une valeur d'énergie de sortie à une longueur d'onde qui, parmi la pluralité de longueurs d'onde, affiche une valeur d'énergie de sortie la plus petite soit supérieure ou égale à 90 % d'une valeur d'énergie de sortie à une longueur d'onde qui, parmi la pluralité de longueurs d'onde, affiche une valeur d'énergie de sortie la plus grande.

5. Appareil laser selon la revendication 4, dans lequel l'organe de commande effectue une commande telle que la valeur d'énergie de sortie à la longueur d'onde qui, parmi la pluralité de longueurs d'onde, affiche la valeur d'énergie de sortie la plus petite soit supérieure ou égale à 95 % de la valeur d'énergie de sortie à la longueur d'onde qui, parmi la pluralité de longueurs d'onde, affiche la valeur d'énergie de sortie la plus grande.

6. Appareil laser selon l'une quelconque des revendications 1 à 5, comprenant en outre un régulateur configuré pour régler et maintenir, à un niveau prédéterminé, une tension appliquée à la lampe à éclair tandis que l'objet est exposé au rayonnement de la lumière laser.

7. Appareil laser selon la revendication 6, dans lequel le régulateur mesure l'énergie de sortie de la lampe à éclair avant que l'objet ne soit exposé au rayonnement de la lumière laser, et augmente la tension appliquée lorsque que l'énergie de sortie ne parvient pas à être obtenue.

8. Appareil laser selon l'une quelconque des revendications 1 à 7, dans lequel le miroir de sortie a une réflectance de lumière qui varie conformément à la longueur d'onde de la lumière laser.

9. Appareil laser selon l'une quelconque des revendications 1 à 8, dans lequel l'oscillateur est un commutateur de Q.

10. Appareil d'acquisition d'informations d'objet, comprenant :
l'appareil laser selon l'une quelconque des revendications 1 à 9,
une sonde (106) configurée pour détecter une onde acoustique générée dans un objet exposé au rayonnement de la lumière laser ; et
un processeur (108) configuré pour acquérir des informations caractéristiques concernant l'objet au moyen de l'onde acoustique.

11. Appareil d'acquisition d'informations d'objet selon la revendication 10, dans lequel :
le sélecteur sélectionne la longueur d'onde de la lumière laser parmi deux longueurs d'onde, et
le processeur calcule une saturation en oxygène de l'objet au moyen des informations caractéristiques obtenues à chacune des deux longueurs d'onde.

12. Appareil d'acquisition d'informations d'objet selon la revendication 11, dans lequel les deux longueurs d'onde sont de 750 nm et de 800 nm.

13. Appareil d'acquisition d'informations d'objet selon l'une quelconque des revendications 10 à 12, comprenant en outre :
un illuminateur qui expose l'objet au rayonnement de la lumière laser ; et
un dispositif à balayage qui anime l'illuminateur d'un mouvement de va-et-vient sur une bande sur l'objet,
dans lequel le sélecteur sélectionne des longueurs d'onde différentes pour un balayage vers l'avant et pour un balayage vers l'arrière à l'intérieur de la bande.

14. Appareil d'acquisition d'informations d'objet selon l'une quelconque des revendications 10 à 13, comprenant en outre un afficheur qui affiche des informations caractéristiques concernant l'objet.
